# EUROPÄISCHE PATENTSCHRIFT

(11) **EP 4 319 680 B1**
(45) Veröffentlichungstag und Bekanntmachung des Hinweises auf die Patenterteilung: **06.08.2025**
(21) Anmeldenummer: 22713848.4
(22) Anmeldetag: 22.03.2022
(51) Int. Cl.: A61C 8/00, A61C 1/00

(54) **VORRICHTUNG ZUR VERWENDUNG BEI EINEM CRESTALEN SINUSLIFT**
DEVICE FOR USE IN A CRESTAL SINUS LIFT
DISPOSITIF DESTINÉ À ÊTRE UTILISÉ DANS UNE ÉLÉVATION DE CRÊTE DE SINUS

(30) Priorität: 09.04.2021 AT 502622021
(43) Veröffentlichungstag der Anmeldung: 14.02.2024
(73) Patentinhaber: Eder, Klaus, 1130 Wien (AT)
(72) Erfinder: Eder, Klaus, 1130 Wien (AT)
(74) Vertreter: Schwarz & Partner Patentanwälte GmbH
(86) Internationale Anmeldenummer: PCT/AT2022/060082
(87) Internationale Veröffentlichungsnummer: WO 2022/213134

(56) Entgegenhaltungen:
- WO-A1-2010/048648
- WO-A1-2014/000007
- JP-A- 2014 087 633
- US-A1- 2016 081 775

## Beschreibung

Die Erfindung betrifft eine Vorrichtung zur Verwendung bei einem crestalen Sinuslift, die Vorrichtung umfassend eine Bohreinrichtung, die dazu ausgebildet ist, Knochenmaterial mittels eines Bohrkopfs abzutragen, und eine Spüleinrichtung, welche die Bohreinrichtung zumindest teilweise aufnimmt und eine Bohröffnung für den Durchtritt des Bohrkopfs aufweist, wobei die Spüleinrichtung dazu ausgebildet ist, eine Flüssigkeit mit einem vorbestimmten Druck durch die Bohröffnung auszugeben, und wobei die Vorrichtung von einer ersten Stellung, in der der Bohrkopf innerhalb der Spüleinrichtung aufgenommen ist ohne durch die Bohröffnung aus der Spüleinrichtung hinauszuragen, in eine zweite Stellung, in der der Bohrkopf durch die Bohröffnung aus der Spüleinrichtung hinausragt, bringbar ist. Bei einem crestalen Sinuslift soll künstliches Knochenmaterial zwischen den Kieferknochen und die Kieferknochenschleimhaut eingebracht werden, um den Kieferknochen zu verstärken. Sobald das künstliche Knochenmaterial ausgehärtet ist, was in der Regel mehrere Monate dauert, kann in diesem Bereich ein Implantat am Kieferknochen befestigt werden, der in seiner natürlichen Form nicht die hierfür ausreichende Stärke aufweisen würde.

Zu diesem Zweck wird ein Kanal in den Kieferknochen eingebohrt, um das künstliche Knochenmaterial zwischen den Kieferknochen und die Kieferknochenschleimhaut einzubringen. Bei der Bohrung ist insbesondere darauf zu achten, dass die äußerst dünne Kieferknochenschleimhaut nicht verletzt wird, da in derartigen Fällen das Verfahren bis zur vollständigen Heilung der Kieferhöhlenschleimhaut unterbrochen werden muss.

Aus dem Stand der Technik (z.B. der DE 10 2007 059 226 B4) ist zu diesem Zweck bekannt, den Kieferknochen anzubohren, bis dieser nur mehr eine vorbestimmte Restdicke aufweist. Danach, oder bereits während des Bohrens, kann eine Kochsalzlösung druckbeaufschlagt in den Hohlraum zwischen Bohrer und abzutragendem Kieferknochen eingebracht werden, sodass sich die Restdicke des Kieferknochens bereits vor dem Auftreffen des Bohrers auf die Kieferhöhlenschleimhaut mit dieser vom Kieferknochen ablöst, wodurch der Hohlraum für das künstliche Knochenmaterial gebildet wird.

Gemäß dem Stand der Technik wird die Bohrung mittels zweier gesonderter Instrumente durchgeführt, einerseits mit dem Bohrer an sich und andererseits mit einer Spüleinrichtung, durch welche der Bohrer üblicherweise durchgeführt ist. Diese Spüleinrichtung ist derart ausgebildet, dass die Kochsalzlösung zielgerecht auf die genannte Restdicke gerichtet werden kann.

In der Praxis stellte sich jedoch heraus, dass derartige Bohrungen vom behandelnden Arzt nur äußerst umständlich vorgenommen werden können, da dieser mit einer Hand den Bohrer bedienen und gleichzeitig mit der anderen Hand die Spüleinrichtung führen muss. Es ist dem Arzt während der Bohrung somit nicht möglich, weitere Behandlungstätigkeiten vorzunehmen, die eine freie Hand benötigen würden.

Aus der JP 2014087633 A ist eine Vorrichtung bekannt, welche eine Bohreinrichtung und eine Spüleinrichtung aufweist, wobei die Spüleinrichtung die Bohreinrichtung zumindest teilweise aufnimmt und eine Feder zwischen den beiden Einrichtungen vorgesehen ist. Weiters weist die Spüleinrichtung eine optische Skala auf, um dem Benutzer die aktuelle Bohrtiefe anzuzeigen.

Die KR 102082212 B1 offenbart eine Bohrvorrichtung zur Bohrung am Alveolarknochen.

Diese Vorrichtung offenbart jedoch keine integrierte Spüleinrichtung, da dem Bohrkopf Flüssigkeit nur von außen durch Löcher in einem den Bohrer umgebenden Element zugeführt wird.

Weitere Bohreinrichtungen sind aus den Druckschriften US 2017/105812 A1 und WO 2014/000007 A1 bekannt.

Es ist daher eine Aufgabe der Erfindung, eine Vorrichtung zur Verwendung bei einem crestalen Sinuslift zu schaffen, die leichter zu bedienen ist als jene des Standes der Technik.

Der Gegenstand der Erfindung ist in den unabhängigen Ansprüchen 1, 2 und 3 definiert.

Die Aufgabe wird gemäß der Erfindung durch die eingangs erwähnte Vorrichtung gelöst, die ferner ein Spannelement umfasst, welches dazu ausgebildet ist, die Vorrichtung in die erste Stellung vorzuspannen und eine Kraft entgegenzusetzen, wenn die Vorrichtung von der ersten Stellung in die zweite Stellung gebracht wird.

Das Spannelement bewirkt erstens, dass durch das Vorspannen in die erste Stellung eine Ruhestellung der Vorrichtung geschaffen wird, sodass die Bohreinrichtung und die Spüleinrichtung als Einheit, d.h. mit einer Hand, handgehabt werden können. Da in der ersten Stellung der Bohrkopf vollständig in der Spüleinrichtung aufgenommen ist, wird zusätzlich eine Sicherung geschaffen, da der Bohrkopf in dieser Stellung selbst bei unbeabsichtigtem Anschalten nicht aus der Bohröffnung herausragt.

Zweitens schafft das Spannelement die Möglichkeit, dass auch während der Bohrung, d.h. nachdem die Vorrichtung aus ihrer Ruheposition heraus bewegt wird, die einhändige Bedienung der Vorrichtung gewährleistet wird, da die Bohreinrichtung nach dem Verlassen der Ruhestellung automatisch in diese rückgeführt wird. Wenn der behandelnde Arzt somit den Bohrkopf in den anzubohrenden Kieferknochen drückt, setzt das Spannelement diesem Anpressen, d.h. dem Versetzen der Vorrichtung von der ersten Stellung in die zweite Stellung, eine Kraft entgegen. Wenn der behandelnde Arzt den Bohrkopf aus dem Kieferknochen extrahieren will, muss dieser lediglich den Anpressdruck verringern. Selbst beim Bohrvorgang ist somit keine zweite Hand notwendig, um den Bohrkopf aus dem gebohrten Loch im Kieferknochen herauszuführen.

Besonders vorteilhaft ist allerdings, dass durch die Verwendung des Spannelements beim Andrücken der Vorrichtung in der ersten Stellung geprüft werden kann, ob durch das Andrücken der Spüleinrichtung an den Kieferknochen bzw. einer darauf befindlichen Haut eine dichte Verbindung der Spüleinrichtung gewährleistet ist, und erst beim Vorliegen der dichten Verbindung weitere Schritte zum Bohren gesetzt werden können. Hierdurch ist eine zuverlässige Spülung während dem Bohren und ein sicheres Loslösen der Kieferknochenschleimhaut vom restlichen Kieferknochen vor dem Durchbohren gewährleistet.

Ein weiterer Vorteil, den das Spannelement mit sich bringt, ist, dass ein vorbestimmter Anpressdruck aufgebracht wird, wenn die Vorrichtung von der ersten Stellung in die zweite Stellung gebracht werden soll. Dies führt dazu, dass die Bohröffnung bei der Bohrung mit dem genannten Anpressdruck am Kieferknochen anliegt, sodass die Spüleinrichtung bei der Bohrung dicht mit dem Kieferknochen verbunden ist.

Für den Beginn der Bohrung kann die Spüleinrichtung somit einfach mit der Bohröffnung an den anzubohrenden Kieferknochen angesetzt werden, während sich die Vorrichtung in der ersten Stellung befindet. Beispielsweise kann die Vorrichtung an einem bereits zuvor angebohrten Sackloch angesetzt werden, um die Orientierung zu erleichtern. Danach wird die Vorrichtung durch einen Anpressdruck von der ersten Stellung in die zweite Stellung gebracht, wobei die Spüleinrichtung weiterhin am Kieferknochen verbleibt und die Bohreinrichtung in den Kieferknochen eindringt, um das Knochenmaterial abzutragen. Gleichzeitig bringt die Spüleinrichtung die druckbeaufschlagte Flüssigkeit in das Bohrloch ein. Sobald der Kieferknochen nur mehr eine Restdicke aufweist, wird diese samt der Kieferhöhlenschleimhaut durch den Druck der Flüssigkeit vom restlichen Kieferknochen abgelöst, bevor der Bohrkopf die Kieferhöhlenschleimhaut verletzt. Danach kann die Bohreinrichtung durch einfaches Verringern des Anpressdrucks aus dem Bohrloch geführt werden, ohne dass hierfür eine weitere Handbewegung nötig wäre.

In einer erfindungsgemäßen Ausführungsform ist die Vorrichtung dazu ausgebildet, den Bohrkopf zur Bohrung anzutreiben, sobald ein vorbestimmter Anpressdruck beim Versetzen von der ersten Stellung in die zweite Stellung überschritten wird und/ oder sobald der Bohrkopf eine vorbestimmte Entfernung zur Bohröffnung unterschreitet. Dies ermöglicht, dass auch das Ein- und Ausschalten des Bohrers automatisch durchgeführt wird. Ein eigener Ein- und Ausschalter für den Bohrer kann somit entfallen auch, wenn dieser beispielsweise aus Gründen der Sicherheit weiterhin vorhanden sein kann. Insbesondere ist in dieser Ausführungsform nicht notwendig, dass der Bohrer mittels der zweiten Hand oder eines Fußpedals ein- bzw. ausgeschaltet werden muss.

Ferner ist erfindungsgemäß vorgesehen, wenn zusätzlich oder alternativ zu der vorgenannten Ausführungsform die Vorrichtung dazu ausgebildet ist, die genannte Flüssigkeit auszugeben, sobald ein vorbestimmter weiterer Anpressdruck beim Versetzen von der ersten Stellung in die zweite Stellung überschritten wird und/ oder wenn der Bohrkopf eine vorbestimmte weitere Entfernung zur Bohröffnung unterschreitet. Hierdurch wird erreicht, dass auch für die Spülung kein gesonderter Ein- bzw. Ausschalter vorgesehen werden muss, was die Bedienung der vorliegenden Vorrichtung weiter vereinfacht. Auch hier könnte ein Schalter zusätzlich zur automatischen Ein- bzw. Ausbringung der Flüssigkeit vorgesehen sein, um die Sicherheit zu erhöhen.

In einer besonders bevorzugten Ausführungsform ist die Vorrichtung dazu ausgebildet, den Druck der Flüssigkeit zu messen und den Bohrkopf zur Bohrung anzutreiben, sobald der gemessene Druck über einem vorbestimmten Schwellwert liegt. Dadurch kann gemessen werden, ob die Bohröffnung dicht am Kieferknochen bzw. einer darauf befindlichen Haut anliegt, bevor der Bohrkopf angetrieben wird. Somit kann eine zusätzliche Sicherheitseinrichtung geschaffen werden, um eine frühzeitige Bohrung zu verhindern. Dies ist insbesondere in Verbindung mit der vorgenannten Ausführungsform vorteilhaft, wenn die Flüssigkeit erst bei einem weiteren Anpressdruck bzw. bei einer weiteren Entfernung ausgegeben wird, da dadurch ein zweistufiges Sicherheitssystem erzielt wird, bei dem erst die Flüssigkeitsausgabe reguliert wird, danach gemessen wird, ob die Bohröffnung dicht anliegt, und erst zuletzt der Bohrkopf angetrieben wird.

Bevorzugt ist das Spannelement eine Feder, wodurch sich die erfinderische Vorrichtung in einfacher Weise erzielen lässt. Insbesondere kann die Feder eine Sprungfeder aus Metall, beispielsweise aus rostfreiem Stahl gefertigt sein, um die hygienischen Standards für medizinische Instrumente zu gewährleisten.

In einer Ausführungsform haben sowohl die Bohreinrichtung als auch die Spüleinrichtung einen eigenen gesonderten Griff, wobei das Spannelement die Griffe zueinander in einer vorbestimmten Relation hält. Die beiden Griffe könnten beispielsweise wie Zangen- oder Scherengriffe ausgebildet sein, um die einhändige Bedienung zu erleichtern.

Bevorzugt ist jedoch, wenn die Vorrichtung nur einen Griff aufweist, wobei der Griff starr an der Bohreinrichtung ausgeführt ist und die Spüleinrichtung mittels des Spannelements versetzbar an der Bohreinrichtung angebracht ist. Dies erlaubt eine konstruktiv besonders einfache Vorrichtung zur Verwendung bei einem crestalen Sinuslift. In dieser Ausführungsform kann ein aus dem Stand der Technik bekannter Bohrer derart erweitert werden kann, dass im Bereich des Bohrkopfes die Spüleinrichtung beweglich vorgesehen wird, wobei das Spannelement die Verbindung zwischen Bohreinrichtung und Spüleinrichtung darstellt.

Vorteilhafte und nicht einschränkende Ausführungsformen der Erfindung werden nachfolgend anhand der Zeichnungen näher erläutert.
Fig. 1A zeigt einen Bohrvorgang bei einem crestalen Sinuslift, bevor der Kieferknochen vollständig durchbohrt ist. Fig. 1B zeigt den genannten Bohrvorgang, nachdem eine Restdicke des Kieferknochens gemeinsam mit der Kieferhöhlenschleimhaut vom Kieferknochen durch den Druck einer eingebrachten Flüssigkeit abgelöst ist.
Fig. 2 zeigt eine Vorrichtung zur Verwendung bei einem crestalen Sinuslift gemäß dem Stand der Technik.
Fig. 3 zeigt die erfindungsgemäße Vorrichtung zur Verwendung bei einem crestalen Sinuslift.
Fig. 4 zeigt die Vorrichtung von Fig. 3 in einer ersten Stellung.
Fig. 5 zeigt die Vorrichtung von Fig. 3 in einer zweiten Stellung.

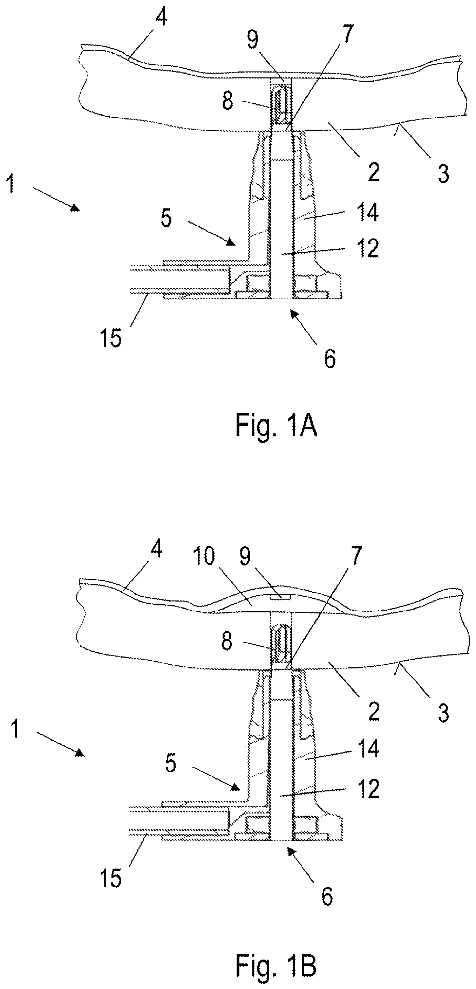

Fig. 1A zeigt eine Vorrichtung 1 zur Verwendung bei einem crestalen Sinuslift. Für einen crestalen Sinuslift wird ein Kieferknochen 2, beispielsweise eines Menschen, ausgehend von einer Bohrseite 3 durchbohrt, um künstliches Knochenmaterial zwischen den Kieferknochen 2 und einer der Bohrseite 3 gegenüberliegenden Kieferknochenschleimhaut 4 einzubringen.

Um die Kieferknochenschleimhaut 4 nicht zu verletzen, darf eine Bohreinrichtung 5 der Vorrichtung 1 nicht zu weit in den Kieferknochen 2 eindringen. Zu diesem Zweck umfasst die Vorrichtung 1 neben der Bohreinrichtung 5 eine davon gesonderte Spüleinrichtung 6. Die Spüleinrichtung 6 weist dabei eine im Wesentlichen zylindrische Ausnehmung auf, in welcher die Bohreinrichtung 5 zumindest teilweise aufgenommen ist. Endseitig weist die Spüleinrichtung 6 eine Bohröffnung 7 auf, durch welche ein Bohrkopf 8 der Bohreinrichtung 5 hindurchtreten kann.

Die Spüleinrichtung 6 verbleibt während des Bohrvorgangs im Wesentlichen in ihrer Position, d.h. an der Bohrseite 3 des Kieferknochens 2 anliegend, entweder direkt am Knochen 3 oder an einer an der Bohrseite 3 befindlichen Haut. Der Bohrkopf 8 dringt hingegen in den Kieferknochen 2 ein und trägt dabei mittels des Bohrkopfs 8 Knochenmaterial ab. Hierfür kann der Bohrkopf 8 beispielsweise in an sich bekannter Weise mit einer einheitlichen oder veränderlichen Drehgeschwindigkeit um eine Drehachse rotieren.

Während des Bohrvorgangs bringt die Spüleinrichtung 6 eine Flüssigkeit, beispielsweise eine Kochsalzlösung, mit einem vorbestimmten Druck, beispielsweise 0,5 - 2 bar, bevorzugt im Wesentlichen 1,5 bar, durch die Bohröffnung 7 auf das abzutragende Knochenmaterial auf. Dies hat den Zweck, dass einerseits abgetragenes Knochenmaterial vom Bohrkopf 8 abtransportiert werden kann. Andererseits hat das Druckbeaufschlagen mittels der Flüssigkeit den Effekt, dass eine Restdicke 9 des Kieferknochens 2 inklusive der Kieferknochenschleimhaut 4 vom restlichen Kieferknochen 2 abgelöst werden kann, bevor der Bohrkopf 8 die Kieferknochenschleimhaut 4 verletzt, siehe Fig. 1B. Dadurch ergibt sich ein Hohlraum 10 zwischen Kieferknochen 2 und Kieferknochenschleimhaut 4, in den folglich das künstliche Knochenmaterial eingebracht werden kann.

Das Ausgeben der Flüssigkeit durch die Spüleinrichtung kann entweder statisch erfolgen, d.h. die Flüssigkeit wird erst nach dem Bohrvorgang aus der Bohröffnung abgeleitet, oder dynamisch, d.h. bereits während des Bohrvorgangs wird kontinuierlich Flüssigkeit durch die Bohröffnung 7 eingebracht und aus dieser ausgebracht.

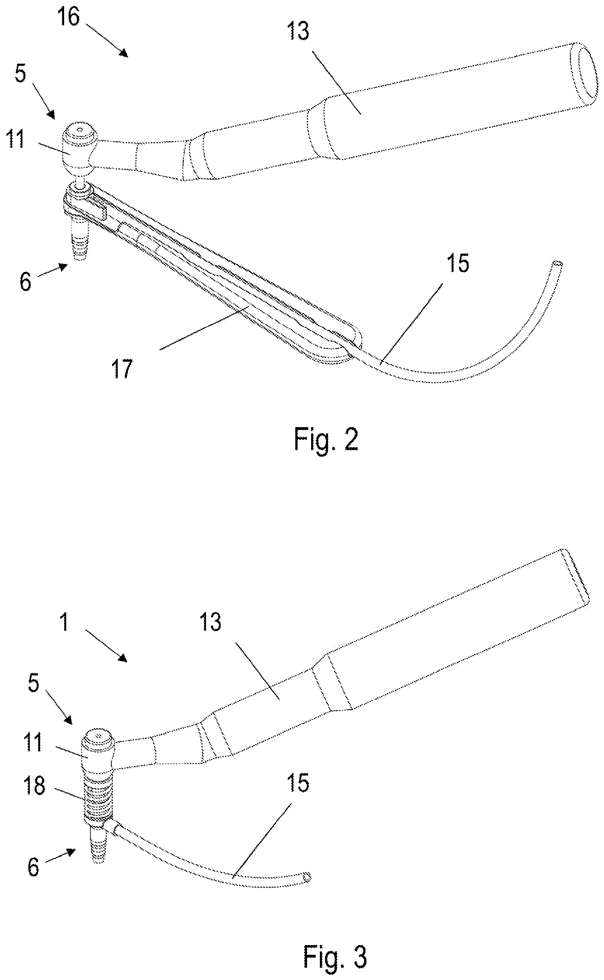

Wie in Fig. 1 in Verbindung mit Fig. 3 gezeigt, umfasst die Bohreinrichtung 5 beispielsweise neben dem Bohrkopf 8 einen Antrieb 11 für den Bohrkopf 8 und eine Führung 12, die den Bohrkopf 8 mit dem Antrieb 11 verbindet. Eine derartige Bohreinrichtung 5 wird üblicherweise an einem am Antrieb 11 ansetzenden Griff 13 manuell geführt.

Die Spüleinrichtung 6 umfasst in der Regel ein Dichtrohr 14 mit der Bohröffnung 7. Das Dichtrohr 14 weist beispielsweise eine zylindrische oder konische Ausnehmung zur Aufnahme des Bohrkopfs 8 bzw. der Führung 12 auf. Die Flüssigkeitszufuhr kann über einen Schlauch 15 erfolgen, der die Flüssigkeit in das Dichtrohr 14 einbringt, sodass die Flüssigkeit zwischen dem Dichtrohr 14 und dem Bohrkopf 8 bzw. der Führung 12 durch die Bohröffnung 7 ausgegeben werden kann. Das Dichtrohr 14 ist auf der der Bohröffnung 7 gegenüberliegenden Seite gegenüber der Führung 12 (bzw. allgemein gegenüber der Bohreinrichtung 5) abgedichtet, sodass die Flüssigkeit nur durch die Bohröffnung 7 die Spüleinrichtung 6 verlässt. Zusätzlich könnte eine Rückführleitung vorgesehen sein, mit der in die Bohröffnung 7 zurückströmende Flüssigkeit wieder rückgeführt werden kann (nicht dargestellt).

Gemäß dem Stand der Technik, der in Fig. 2 durch eine Vorrichtung 16 beispielhaft dargestellt ist, sind die Bohreinrichtung 5 und die Spüleinrichtung 6 als gesonderte Instrumente ausgeführt. Hier wird die Spüleinrichtung 6 an einem am Dichtrohr 14 ansetzenden Griff 17 manuell geführt. Dies bedeutet in der Praxis, dass der den Bohrvorgang vornehmende Arzt beide Hände verwenden muss, um die Bohrung für den crestalen Sinuslift durchzuführen.

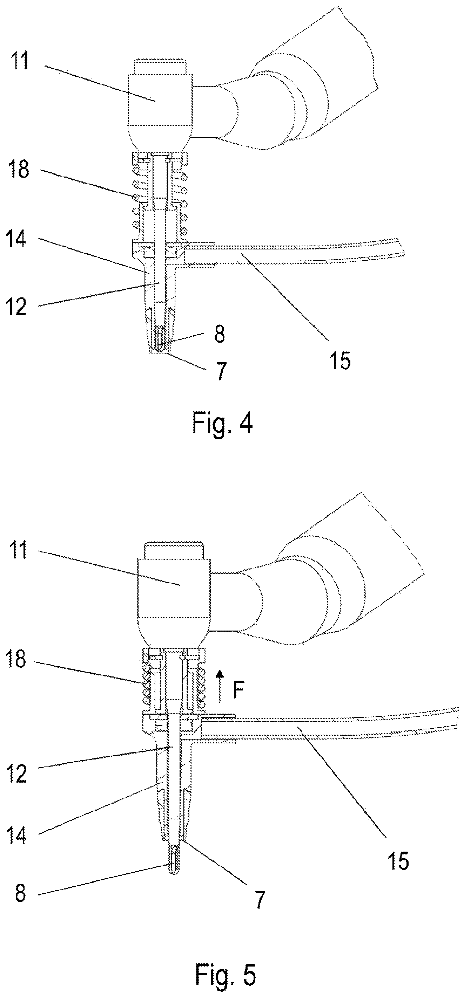

Fig. 3 zeigt die erfindungsgemäße Vorrichtung 1, bei der die Bohreinrichtung 5 und die Spüleinrichtung 6 mittels eines Spannelements 18 verbunden sind. Das Spannelement 18 spannt die Vorrichtung 1 in eine erste Stellung (Fig. 4) vor, d.h. in eine Stellung, in der der Bohrkopf 8 innerhalb der Spüleinrichtung 6 aufgenommen ist ohne durch die Bohröffnung 7 aus der Spüleinrichtung 6 hinauszuragen. In dieser Ruhestellung ist der Bohrkopf 8 selbst bei einem unabsichtlichen Einschalten der Bohreinrichtung 5 durch die Spüleinrichtung 6 abgesichert, da er nicht aus der Bohröffnung 7 herausragt.

Wenn die Vorrichtung 1 mittels eines Anpressdrucks in eine zweite Stellung (Fig. 5) verbracht wird, in der der Bohrkopf 8 durch die Bohröffnung 7 aus der Spüleinrichtung 6 hinausragt, setzt das Spannelement 18 diesem Versatz eine Kraft F entgegen, sodass die Vorrichtung 1 wieder in die erste Stellung gebracht wird, sofern der Arzt den Anpressdruck beim Bohren nicht aufrechthält. Das Spannelement 18 kann beispielsweise derart gewählt werden, dass die Vorrichtung 1 bei einem Einwirken von 1 kg - 3,5 kg, bevorzugt im Wesentlichen 2,5 kg, von der ersten Stellung vollständig in die zweite Stellung gebracht wird.

In der ersten Stellung weist der Bohrkopf 8, d.h. eine endseitige Spitze des Bohrkopfs 8, innerhalb der Spüleinrichtung 6 beispielsweise eine Entfernung von 0,1 - 2 mm zur Bohröffnung 7 auf. Die Spitze kann jedoch auch in der Bohröffnung 7 liegen. In der zweiten Stellung liegt die Spitze des Bohrkopfs 8 außerhalb der Spüleinrichtung 6 und hat eine Entfernung zur Bohröffnung 7, die beispielsweise im Wesentlichen der zu bohrenden Kieferknochendicke entspricht.

Durch das Spannelement 18 kann die Vorrichtung 1 somit während des Bohrvorgangs auch einhändig bedient werden, da die Bohreinrichtung 5 und die Spüleinrichtung 6 mittels einer geeigneten elastischen Verbindung gekoppelt sind.

Das Spannelement 18 kann beispielsweise eine Feder sein, um die genannte Funktion zu erfüllen, und beispielsweise aus rostfreiem Stahl gefertigt sein. Alternativ könnte das Spannelement 18 als Kunststoffring oder anderes mechanisches Element mit geeigneten elastischen Eigenschaften ausgebildet sein. Ferner könnte das Spannelement 18 auch durch geeignete magnetische oder elektromagnetische Elemente erzielt werden.

Gemäß Fig. 3 weist die Vorrichtung 1 nur einen Griff 13 auf, wobei der Griff 13 starr an der Bohreinrichtung 5 ausgeführt ist und die Spüleinrichtung 6 mittels des Spannelements 18 versetzbar an der Bohreinrichtung 5 angebracht ist. Die Spüleinrichtung 6 weist in dieser Ausführungsform keinen gesonderten Griff auf, wodurch die Vorrichtung 1 einfach handhabbar ist. Die Spülvorrichtung 6 kann in dieser Ausführungsform mit dem Spannelement 18 als Aufsatz auf im Handel erhältliche Bohrer montiert werden, um die Vorrichtung 1 zu erhalten.

In einer alternativen Ausführungsform (nicht dargestellt) hat auch die Spüleinrichtung 6 einen Griff, der beispielsweise zu dem Griff 13 der Bohreinrichtung 5 komplementär ist, wie dies von Scheren oder Zangen bekannt ist. Weiterhin verbindet das Spannelement 18 jedoch die Bohreinrichtung 5 und die Spüleinrichtung 6 in der genannten Weise, sodass auch hier eine einhändige Bedienung gewährleistet ist.

Im einfachsten Fall weist die Vorrichtung 1 am Griff 13 oder an einem anderen Griff einen Ein- und Ausschalter, z.B. einen Druckschalter, auf, um den Bohrkopf 8 zur Bohrung anzutreiben und einen weiteren Ein- und Ausschalter, um die Flüssigkeit aus der Spüleinrichtung 6 auszugeben. Es könnte auch ein Ein- und Ausschalter vorgesehen werden, der gleichzeitig den Antrieb des Bohrkopfs 8 und die Ausgabe der Flüssigkeit steuert.

Um den Beginn der Bohrung ohne Schalter steuern zu können, treibt die Vorrichtung 1 den Bohrkopf 8 zur Bohrung an, sobald ein vorbestimmter Anpressdruck beim Versetzen von der ersten Stellung in die zweite Stellung überschritten wird und/ oder sobald der Bohrkopf 8 eine vorbestimmte Entfernung zur Bohröffnung 7 unterschreitet. Zur Messung des Drucks bzw. der Entfernung können verschiedene Sensoren eingesetzt werden, beispielsweise ein piezoelektrisches Element zur Messung des Drucks oder ein induktives Element zur Messung der Entfernung. Zwar ist in dieser Ausführungsform kein gesonderter Schalter zwingend, jedoch kann ein solcher aus Sicherheitsgründen beispielsweise weiterhin am Griff 13 der Bohreinrichtung 5 angebracht werden, z.B. als Druckschalter.

Alternativ oder zusätzlich kann die Vorrichtung 1 die genannte Flüssigkeit ausgeben, sobald ein vorbestimmter weiterer Anpressdruck beim Versetzen von der ersten Stellung in die zweite Stellung überschritten wird und/ oder wenn der Bohrkopf 8 eine vorbestimmte weitere Entfernung zur Bohröffnung 7 unterschreitet. Auch hier kann zusätzlich ein Schalter am Griff 13 oder einem anderen Griff angebracht sein.

Der genannte vorbestimmte Anpressdruck bzw. der genannte vorbestimmte weitere Anpressdruck liegt jeweils über der Vorspannkraft des Spannelements 18. Die genannte vorbestimmte Entfernung bzw. die vorbestimmte weitere Entfernung kann entweder - relativ zur Bohröffnung 7 - innerhalb oder außerhalb der Spüleinrichtung 6 liegen. Beispielsweise kann die Entfernung bzw. die weitere Entfernung derart gewählt werden, dass der Bohrkopf 8 angetrieben oder die Flüssigkeit ausgegeben wird, bevor oder nachdem der Bohrkopf 8 die Bohröffnung 7 durchtritt.

Wenn sowohl der Antrieb des Bohrkopfs 8 als auch die Ausgabe der Flüssigkeit wie oben beschrieben schalterlos erfolgen, können bereits zwei Betriebsknöpf - einer zum Antrieb des Bohrkopfs 8 und einer zur Ausgabe der Flüssigkeit - eingespart werden. In dieser Ausführungsform kann der vorbestimmte weitere Anpressdruck bzw. die vorbestimmte weitere Entfernung zur Ausgabe der Flüssigkeit identisch oder unterschiedlich zum vorbestimmten Anpressdruck bzw. zur vorbestimmten Entfernung zur Aktivierung der Bohrung gewählt werden. Beispielsweise kann der Bohrkopf 8 bereits angetrieben werden, bevor dieser die Bohröffnung 7 durchschreitet, und die Flüssigkeit kann erst ausgegeben werden, nachdem der Bohrkopf 8 die Bohröffnung 7 passiert hat.

Eine weitere Steuerung der Flüssigkeitsausgabe und des Antriebs des Bohrkopfs 8 kann wie folgt erfolgen. Zuerst wird - bei ruhendem Bohrkopf 8 - die Flüssigkeit ausgegeben, entweder durch gesonderten Knopfdruck bzw. Fußschalter oder automatisch, wenn wie oben beschrieben ein vorbestimmter weiterer Anpressdruck beim Versetzen von der ersten Stellung in die zweite Stellung überschritten wird und/ oder wenn der Bohrkopf 8 eine vorbestimmte weitere Entfernung zur Bohröffnung 7 unterschreitet. Nach der Ausgabe der Flüssigkeit wird der Druck der Flüssigkeit gemessen, d.h. es wird überprüft, ob die Bohröffnung dicht am Kieferknochen anliegt. Sollte dies nicht der Fall sein, tritt die Flüssigkeit seitlich zwischen Bohröffnung 7 und Kieferknochen aus und es kommt zu einem Druckabfall, der unmittelbar gemessen werden kann. Erst nachdem sichergestellt wurde, dass der gemessene Druck über einem vorbestimmten Schwellwert liegt, wird der Bohrkopf 8 zur Bohrung angetrieben. Hier kann zusätzlich zur Messung des Flüssigkeitsdrucks vorgesehen werden, dass auch der Anpressdruck erreicht wurde und/ oder der Bohrkopf 8 die vorbestimmte Entfernung zur Bohröffnung 7 unterschritten hat, bevor der Bohrkopf 8 angetrieben wird.

In dieser Ausführungsform kann die Flüssigkeit beispielsweise ausgegeben werden, wenn 1 kg auf das Spannelement 18 einwirkt und der Bohrkopf 8 kann angetrieben werden, wenn ein Schwellwert für den Druck der Flüssigkeit von 0,8 bar erreicht wird. Zur Messung des Drucks der Flüssigkeit kann ein Druckmesser in der Spüleinrichtung 6 angeordnet sein, beispielsweise innerhalb des Dichtrohres 14 oder in einer Flüssigkeitszufuhr, die an einer der dem Dichtrohr 14 abgewandten Seite des Schlauchs 15 angeordnet ist.

Die Abschaltung des Antriebs des Bohrkopfs 8 bzw. die Abschaltung der Ausgabe der Flüssigkeit kann erfolgen, wenn der genannte vorbestimmte (weitere) Anpressdruck wieder unterschritten wird bzw. die vorbestimmte (weitere) Entfernung wieder überschritten wird, d.h. wenn der Bohrkopf 8 wieder in die Spüleinrichtung 6 eingefahren wird. Alternativ können auch andere Schwellwerte vorgesehen werden. So kann beispielsweise die Abschaltung des Antriebs des Bohrkopfs 8 bzw. die Abschaltung der Ausgabe der Flüssigkeit erst dann erfolgen, wenn sich die Vorrichtung 1 wieder in ihrer ersten Stellung befindet, d.h. wenn die Entfernung des Bohrkopfs 8 von der Bohröffnung 7 innerhalb der Spüleinrichtung 6 maximal ist bzw. wenn kein Anpressdruck vorhanden ist. Dies verhindert ein vorzeitiges, ungewolltes Abschalten des Antriebs des Bohrkopfs bzw. der Ausgabe der Flüssigkeit.

## Patentansprüche

1. Vorrichtung (1) zur Verwendung bei einem crestalen Sinuslift, die Vorrichtung (1) umfassend eine Bohreinrichtung (5), die dazu ausgebildet ist Knochenmaterial mittels eines Bohrkopfs (8) abzutragen, und eine Spüleinrichtung (6), welche die Bohreinrichtung (5) zumindest teilweise aufnimmt und eine Bohröffnung (7) für den Durchtritt des Bohrkopfs (8) aufweist,
wobei die Spüleinrichtung (6) dazu ausgebildet ist eine Flüssigkeit mit einem vorbestimmten Druck durch die Bohröffnung (7) auszugeben, und
wobei die Vorrichtung (1) von einer ersten Stellung, in der der Bohrkopf (8) innerhalb der Spüleinrichtung (6) aufgenommen ist ohne durch die Bohröffnung (7) aus der Spüleinrichtung (6) hinauszuragen, in eine zweite Stellung, in der der Bohrkopf (8) durch die Bohröffnung (7) aus der Spüleinrichtung (6) hinausragt, bringbar ist,
wobei ein Spannelement (18) vorgesehen ist, welches dazu ausgebildet ist die Vorrichtung (1) in die erste Stellung vorzuspannen und eine Kraft (F) entgegenzusetzen, wenn die Vorrichtung (1) von der ersten Stellung in die zweite Stellung gebracht wird,
**dadurch gekennzeichnet, dass** die Vorrichtung (1) dazu ausgebildet ist den Bohrkopf (8) zur Bohrung anzutreiben, sobald ein vorbestimmter Anpressdruck beim Versetzen von der ersten Stellung in die zweite Stellung überschritten wird und/ oder sobald der Bohrkopf (8) eine vorbestimmte Entfernung zur Bohröffnung (7) unterschreitet.

2. Vorrichtung (1) zur Verwendung bei einem crestalen Sinuslift, die Vorrichtung (1) umfassend eine Bohreinrichtung (5), die dazu ausgebildet ist Knochenmaterial mittels eines Bohrkopfs (8) abzutragen, und eine Spüleinrichtung (6), welche die Bohreinrichtung (5) zumindest teilweise aufnimmt und eine Bohröffnung (7) für den Durchtritt des Bohrkopfs (8) aufweist,
wobei die Spüleinrichtung (6) dazu ausgebildet ist eine Flüssigkeit mit einem vorbestimmten Druck durch die Bohröffnung (7) auszugeben, und
wobei die Vorrichtung (1) von einer ersten Stellung, in der der Bohrkopf (8) innerhalb der Spüleinrichtung (6) aufgenommen ist ohne durch die Bohröffnung (7) aus der Spüleinrichtung (6) hinauszuragen, in eine zweite Stellung, in der der Bohrkopf (8) durch die Bohröffnung (7) aus der Spüleinrichtung (6) hinausragt, bringbar ist,
wobei ein Spannelement (18) vorgesehen ist, welches dazu ausgebildet ist die Vorrichtung (1) in die erste Stellung vorzuspannen und eine Kraft (F) entgegenzusetzen, wenn die Vorrichtung (1) von der ersten Stellung in die zweite Stellung gebracht wird,
**dadurch gekennzeichnet, dass** die Vorrichtung (1) dazu ausgebildet ist, die genannte Flüssigkeit auszugeben, sobald ein vorbestimmter weiterer Anpressdruck beim Versetzen von der ersten Stellung in die zweite Stellung überschritten wird und/ oder wenn der Bohrkopf (8) eine vorbestimmte weitere Entfernung zur Bohröffnung (7) unterschreitet.

3. Vorrichtung (1) zur Verwendung bei einem crestalen Sinuslift, die Vorrichtung (1) umfassend eine Bohreinrichtung (5), die dazu ausgebildet ist Knochenmaterial mittels eines Bohrkopfs (8) abzutragen, und eine Spüleinrichtung (6), welche die Bohreinrichtung (5) zumindest teilweise aufnimmt und eine Bohröffnung (7) für den Durchtritt des Bohrkopfs (8) aufweist,
wobei die Spüleinrichtung (6) dazu ausgebildet ist eine Flüssigkeit mit einem vorbestimmten Druck durch die Bohröffnung (7) auszugeben, und
wobei die Vorrichtung (1) von einer ersten Stellung, in der der Bohrkopf (8) innerhalb der Spüleinrichtung (6) aufgenommen ist ohne durch die Bohröffnung (7) aus der Spüleinrichtung (6) hinauszuragen, in eine zweite Stellung, in der der Bohrkopf (8) durch die Bohröffnung (7) aus der Spüleinrichtung (6) hinausragt, bringbar ist,
wobei ein Spannelement (18) vorgesehen ist, welches dazu ausgebildet ist die Vorrichtung (1) in die erste Stellung vorzuspannen und eine Kraft (F) entgegenzusetzen, wenn die Vorrichtung (1) von der ersten Stellung in die zweite Stellung gebracht wird,
**dadurch gekennzeichnet, dass** die Vorrichtung (1) dazu ausgebildet ist den Bohrkopf (8) zur Bohrung anzutreiben und die genannte Flüssigkeit auszugeben, sobald ein vorbestimmter Anpressdruck beim Versetzen von der ersten Stellung in die zweite Stellung überschritten wird und/ oder sobald der Bohrkopf (8) eine vorbestimmte Entfernung zur Bohröffnung (7) unterschreitet.

4. Vorrichtung (1) nach Anspruch 2, **dadurch gekennzeichnet, dass** die Vorrichtung (1) dazu ausgebildet ist, den Druck der Flüssigkeit zu messen und den Bohrkopf (8) zur Bohrung anzutreiben, sobald der gemessene Druck über einem vorbestimmten Schwellwert liegt.

5. Vorrichtung (1) nach einem der Ansprüche 1 bis 4, **dadurch gekennzeichnet, dass** das Spannelement (18) eine Feder ist.

6. Vorrichtung (1) nach einem der Ansprüche 1 bis 5, **dadurch gekennzeichnet, dass** die Vorrichtung (1) nur einen Griff (13) aufweist, wobei der Griff (13) starr an der Bohreinrichtung (5) ausgeführt ist und die Spüleinrichtung (6) mittels des Spannelements (18) versetzbar an der Bohreinrichtung (5) angebracht ist.

## Claims

1. Device (1) for use in a crestal sinus lift, the device (1) comprising a drilling means (5) configured to remove bone material by means of a drill head (8), and an irrigation means (6) which at least partially accommodates the drilling means (5) and has a drilling opening (7) for the passage of the drill head (8),
wherein the irrigation means (6) is configured to discharge a liquid at a predetermined pressure through the drilling opening (7), and
wherein the device (1) can be moved from a first position, in which the drill head (8) is accommodated within the irrigation means (6) without projecting out of the irrigation means (6) through the drilling opening (7), into a second position, in which the drill head (8) projects out of the irrigation means (6) through the drilling opening (7),
wherein a tensioning element (18) is provided, which is configured to pretension the device (1) into the first position and to apply a force (F) against it when the device (1) is brought from the first position into the second position,
**characterized in that** the device (1) is configured to drive the drill head (8) for drilling as soon as a predetermined contact pressure is exceeded when moving from the first position to the second position and/or as soon as the drill head (8) falls below a predetermined distance to the drilling opening (7).

2. Device (1) for use in a crestal sinus lift, the device (1) comprising a drilling means (5) configured to remove bone material by means of a drill head (8), and an irrigation means (6) which at least partially accommodates the drilling means (5) and has a drilling opening (7) for the passage of the drill head (8),
wherein the irrigation means (6) is configured to discharge a liquid at a predetermined pressure through the drilling opening (7), and
wherein the device (1) can be moved from a first position, in which the drill head (8) is accomodated within the irrigation means (6) without projecting out of the irrigation means (6) through the drilling opening (7), into a second position, in which the drill head (8) projects out of the irrigation means (6) through the drilling opening (7),
wherein a tensioning element (18) is provided, which is configured to pretension the device (1) into the first position and to apply a force (F) against it when the device (1) is brought from the first position into the second position,
**characterized in that** the device (1) is configured to dispense the said liquid as soon as a predetermined further contact pressure is exceeded when moving from the first position to the second position and/or when the drill head (8) falls below a predetermined further distance to the drilling opening (7).

3. Device (1) for use in a crestal sinus lift, the device (1) comprising a drilling means (5) configured to remove bone material by means of a drill head (8), and an irrigation means (6) which at least partially accommodates the drilling means (5) and has a drilling opening (7) for the passage of the drill head (8),
wherein the irrigation means (6) is configured to discharge a liquid at a predetermined pressure through the drilling opening (7), and
wherein the device (1) can be moved from a first position, in which the drill head (8) is accommodated within the irrigation means (6) without projecting out of the irrigation means (6) through the drilling opening (7), into a second position, in which the drill head (8) projects out of the irrigation means (6) through the drilling opening (7),
wherein a tensioning element (18) is provided, which is configured to pretension the device (1) into the first position and to apply a force (F) against it when the device (1) is brought from the first position into the second position,
**characterized in that** the device (1) is configured to drive the drill head (8) for drilling and to dispense the said liquid as soon as a predetermined contact pressure is exceeded when moving from the first position to the second position and/or as soon as the drill head (8) falls below a predetermined distance to the drilling opening (7).

4. Device (1) according to claim 2, **characterized in that** the device (1) is configured to measure the pressure of the liquid and to drive the drill head (8) for drilling as soon as the measured pressure is above a predetermined threshold value.

5. Device (1) according to one of claims 1 to 4, **characterized in that** the tensioning element (18) is a spring.

6. Device (1) according to one of claims 1 to 5, **characterized in that** the device (1) has only one handle (13), the handle (13) being rigidly provided on the drilling means (5) and the irrigation means (6) being displaceably attached to the drilling means (5) by means of the tensioning element (18).

## Revendications

1. Dispositif (1) destiné à être utilisé pour un sinus lift crestal, le dispositif (1) comprenant un dispositif de forage (5) conçu pour prélever du matériau osseux au moyen d'une tête de forage (8), et un dispositif de rinçage (6) recevant au moins partiellement le dispositif de forage (5) et présentant un trou de forage (7) pour le passage de la tête de forage (8),
dans lequel le dispositif de rinçage (6) est conçu pour émettre un liquide à une pression prédéterminée à travers le trou de forage (7), et
dans lequel le dispositif (1) peut être amené d'une première position, dans laquelle la tête de forage (8) est logée à l'intérieur du dispositif de rinçage (6) sans dépasser du dispositif de rinçage (6) à travers le trou de forage (7), à une deuxième position, dans laquelle la tête de forage (8) dépasse du dispositif de rinçage (6) à travers le trou de forage (7),
dans lequel un élément de tension (18) est prévu qui est conçu pour précontraindre le dispositif (1) dans la première position et pour s'opposer avec une force (F) lorsque le dispositif (1) est amené de la première position à la deuxième position,
**caractérisé en ce que** le dispositif (1) est conçu pour entraîner la tête de forage (8) pour le forage dès qu'une pression de contact prédéterminée est dépassée lors du déplacement de la première position vers la deuxième position et/ou dès que la tête de forage (8) descend en dessous d'une distance prédéterminée du trou de forage (7).

2. Dispositif (1) destiné à être utilisé pour un sinus lift crestal, le dispositif (1) comprenant un dispositif de forage (5) conçu pour prélever du matériau osseux au moyen d'une tête de forage (8), et un dispositif de rinçage (6) recevant au moins partiellement le dispositif de forage (5) et présentant un trou de forage (7) pour le passage de la tête de forage (8),
dans lequel le dispositif de rinçage (6) est conçu pour émettre un liquide à une pression prédéterminée à travers le trou de forage (7), et
dans lequel le dispositif (1) peut être amené d'une première position, dans laquelle la tête de forage (8) est logée à l'intérieur du dispositif de rinçage (6) sans dépasser du dispositif de rinçage (6) à travers le trou de forage (7), à une deuxième position, dans laquelle la tête de forage (8) dépasse du dispositif de rinçage (6) à travers le trou de forage (7),
dans lequel un élément de tension (18) est prévu qui est conçu pour précontraindre le dispositif (1) dans la première position et pour s'opposer avec une force (F) lorsque le dispositif (1) est amené de la première position à la deuxième position,
**caractérisé en ce que** le dispositif (1) est conçu pour émettre ledit liquide dès qu'une autre pression de contact prédéterminée est dépassée lors du déplacement de la première position vers la deuxième position et/ou lorsque la tête de forage (8) descend en dessous d'une autre distance prédéterminée du trou de forage (7).

3. Dispositif (1) destiné à être utilisé pour un sinus lift crestal, le dispositif (1) comprenant un dispositif de forage (5) conçu pour prélever du matériau osseux au moyen d'une tête de forage (8), et un dispositif de rinçage (6) recevant au moins partiellement le dispositif de forage (5) et présentant un trou de forage (7) pour le passage de la tête de forage (8),
dans lequel le dispositif de rinçage (6) est conçu pour émettre un liquide à une pression prédéterminée à travers le trou de forage (7), et
dans lequel le dispositif (1) peut être amené d'une première position, dans laquelle la tête de forage (8) est logée à l'intérieur du dispositif de rinçage (6) sans dépasser du dispositif de rinçage (6) à travers le trou de forage (7), à une deuxième position, dans laquelle la tête de forage (8) dépasse du dispositif de rinçage (6) à travers le trou de forage (7),
dans lequel un élément de tension (18) est prévu qui est conçu pour précontraindre le dispositif (1) dans la première position et pour s'opposer avec une force (F) lorsque le dispositif (1) est amené de la première position à la deuxième position,
**caractérisé en ce que** le dispositif (1) est conçu pour entraîner la tête de forage (8) pour le forage et pour émettre ledit liquide dès qu'une pression de contact prédéterminée est dépassée lors du déplacement de la première position vers la deuxième position et/ou dès que la tête de forage (8) descend en dessous d'une distance prédéterminée du trou de forage (7).

4. Dispositif (1) selon la revendication 2, **caractérisé en ce que** le dispositif (1) est conçu pour mesurer la pression du liquide et pour entraîner la tête de forage (8) pour le forage dès que la pression mesurée est supérieure à une valeur de seuil prédéterminée.

5. Dispositif (1) selon l'une des revendications 1 à 4, **caractérisé en ce que** l'élément de tension (18) est un ressort.

6. Dispositif (1) selon l'une des revendications 1 à 5, **caractérisé en ce que** le dispositif (1) ne comporte qu'une seule poignée (13), la poignée (13) étant réalisée de manière rigide sur le dispositif de forage (5) et le dispositif de rinçage (6) étant monté de manière déplaçable sur le dispositif de forage (5) au moyen de l'élément de tension (18).
